# EUROPÄISCHE PATENTSCHRIFT

(11) **EP 1 872 825 B1**
(45) Veröffentlichungstag und Bekanntmachung des Hinweises auf die Patenterteilung: **11.02.2009**
(21) Anmeldenummer: 07010843.6
(22) Anmeldetag: 01.06.2007
(51) Int. Cl.: A61N 1/05, A61N 1/16, H01B 7/04

(54) **Elektrodeneinrichtung für die Elektrodiagnose und/oder -therapie**
Electrode device for electro-diagnosis and/or electrotherapy
Dispositif a électrodes pour le diagnostic électrique et/ou l'électrothérapie

(30) Priorität: 28.06.2006 DE 102006029864
(43) Veröffentlichungstag der Anmeldung: 02.01.2008
(73) Patentinhaber: BIOTRONIK CRM Patent AG, 6341 Baar (CH)
(72) Erfinder: Flach, Erhard, 12305 Berlin (DE); Geistert, Wolfgang, Dr., 79618 Rheinfelden (DE); Kolberg, Gernot, 12043 Berlin (DE); Maxfield, Michelle, 140967 Berlin (DE); Weiss, Ingo, Dr., 12435 Berlin (DE)
(74) Vertreter: Lindner-Vogt, Karin L.

(56) Entgegenhaltungen:
- EP-A- 1 354 610
- WO-A-20/05053555
- WO-A-20/05116702
- FR-A- 2 446 001
- US-A- 4 198 991
- US-A- 5 433 730
- US-A- 5 554 176
- US-A- 5 683 444
- US-A1- 2005 222 658

## Beschreibung

Die Erfindung betrifft eine Elektrodeneinrichtung für die kardiologische oder neurologische Elektrodiagnose und/oder -therapie mit den im Oberbegriff des Patentanspruches 1 angegebenen Merkmalen.

Derartige Elektrodeneinrichtungen sind beispielsweise aus der WO 2005/053555 A1 bekannt. Sie werden im Bereich der Elektrophysiologie insbesondere zur Erfassung und Behandlung von Reizleitungsstörungen im Herzen oder im Nervensystem eingesetzt und auch als Stimulations-, Schrittmacher- oder ICD-Elektrode bzw. als EP-Katheter (Elektrophysiologie-Katheter) bezeichnet. Sie weisen einen langgestreckten Elektrodenkörper auf, der an oder vor seinem distalen Ende mit mindestens einer Elektrode versehen ist. Bei letzterer kann es sich beispielsweise um eine Sensing-Elektrode zur Erfassung kardiologischer oder neuronaler Signale, eine Ablationselektrode zur lokalen Verödung von Herzgewebe oder eine Therapieelektrode zur Abgabe von elektrischen Reizsignalen beispielsweise eines Neurostimulators, Herzschrittmachers oder Defibrillators handeln. Die Elektrode(n) sind jeweils mit einer Elektrodenleitung für ihre elektrische Anbindung an ein entsprechendes Basisgerät, wie einen elektrischen Generator, ein Elektrotherapiegerät oder ein Implantat, wie einen Neurostimulator, Herzschrittmacher oder Defibrillator, versehen.

Übliche Elektrodeneinrichtungen, wie sie aus dem Stand der Technik in vielfältigen Ausführungsformen bekannt sind, verwenden als Elektrodenleitungen massive, metallische Zuleitungen oder Litzen, bei denen die Einzelleiter nicht voneinander isoliert sind. Ein Patient, der eine derartige Elektrodeneinrichtung implantiert hat, ist von der schonenden Magnetresonanz-Diagnostik mit Hilfe von Kernspintomographen ausgeschlossen, da sich derartige metallische Elektrodenleitungen in extrem starken elektromagnetischen Feldern, wie sie in Magnetresonanz-Tomographen (MRT) auftreten, aufgrund der fließenden Induktionsströme oder die umgebenden Gewebeschichten durch an den Leitungsenden austretende Induktionsströme stark erwärmen können.

Aus der oben genannten WO 2005/053 555 A1 ist es bereits bekannt, die Elektrodenleitung aus Kohlefasern zu bilden, die eine Vielzahl von Filamenten umfassen. Damit lässt sich zwar eine gewisse Verbesserung gegenüber massiven metallischen Zuleitungen oder Litzen erzielen, jedoch erscheinen die Leitungscharakteristika derartiger Elektrodenleitungen im Hinblick auf ihre Einsetzbarkeit in starken Magnetfeldern noch verbesserungsbedürftig.

Aus der US 2005/222658 ist eine Elektrodenleitung für kardiologische oder neurologische Zwecke, welche einen langgestreckten Elektrodenkörper, mehrere Elektroden in der Nähe des distalen Endes des Elektrodenkörpers, eine Elektrodenleitung für die elektrische Anbindung der Elektrode umfasst. Die Elektrodenleitung weist einen Faser-Aufbau, wobei die einzelnen Fasern eine hochohmige bis isolierende Oberflächenschicht aufweisen, welche durch Oxidation des Fasermaterials selbst an der Oberfläche gebildet ist.
Insoweit liegt der Erfindung die Aufgabe zugrunde, eine Elektrodeneinrichtung für die Elektrodiagnose und/oder -therapie auf den Betrieb im MR-Tomographen hin zu optimieren.

Diese Aufgabe wird laut Kennzeichnungsteil des Anspruches 1 dadurch gelöst, dass der Faseraufbau durch die Oberflächenschicht der Fasern eine anisotrope Leitfähigkeit erhält. Die spezifische Leitfähigkeit der Elektrodenleitung ist dabei in ihrer Längsrichtung um mindestens eine Größenordnung höher als in ihrer Querrichtung ist. Die spezifische Leitfähigkeit der Elektrodenleitung ist also in ihrer Längsrichtung signifikant höher als in ihrer Querrichtung. "Signifikant höher" bedeutet in diesem Zusammenhang, dass die Anisotropie der spezifischen Leitfähigkeit abgestimmt auf die Dimensionen der Elektrodenleitung selbst und der Stärke und dem Charakter des bei der MR-Tomographie zum Einsatz kommenden Magnetfeldes so gewählt sein soll, dass bei einem Einsatz der Elektrodeneinrichtung bei der MR-Tomographie keine physiologisch bedenkliche Erwärmung der Elektrodenleitung stattfindet.

der Faser-Aufbau besteht aus einem Bündel von Einzelfasern Letztere sind durch lose aneinanderliegende Filamente gebildet.

Für die Realisierung der anisotropen Leitfähigkeit der Elektrodenleitung ist es besonders effektiv, wenn die Fasern in sich eine anisotrope, in Längsrichtung signifikant höhere Leitfähigkeit als in Querrichtung aufweisen. Dazu kann aufgrund des Herstellungsprozesses jede Einzelfaser so ausgelegt sein, dass ihre Leitfähigkeit aufgrund der intrinsischen Materialeigenschaften radial von innen nach außen graduell abnimmt. Einfacher realisierbar ist jedoch ein Faseraufbau, bei dem eine hochohmige bis isolierende Oberflächenschicht der Faser hergestellt wird. Diese Oberflächenschicht ist durch eine Stoffumwandlung des Fasermaterials selbst gebildet und besteht beispielsweise aus bei der Umwandlung von Polymerfasern in Karbonfasern durch starkes Erhitzen entstehende, hochohmige Residuen. Alternativ dazu kann eine vom eigentlichen Fasermaterial separate Oberflächenschicht durch eine Oberflächenreaktion, wie insbesondere eine Oxidation, des Fasermaterials gebildet werden.

Grundsätzlich eignen sich für die Fasern der Elektrodeneinrichtung alle ausreichend leitfähigen und mechanisch in entsprechende Form bringbare Materialien, wie Kohlenstoff, Metalle, leitende Kunststoffe (wie aus Kondensatoren mit festem Elektrolyt bekannt) oder Halbleitermaterialien.

Weitere Merkmale, Einzelheiten und Vorteile der Erfindung ergeben sich aus der nachfolgenden Beschreibung, in der ein Ausführungsbeispiel anhand der beigefügten Zeichnungen näher erläutert wird. Es zeigen:
- Fig. 1: eine Prinzipskizze einer bipolaren Elektrodeneinrichtung mit einem vergrößerten Detailausschnitt der Elektrodenleitung,
- Fig. 2: eine ausschnittsweise Ansicht einer Einzelfaser der Elektrodenleitung gemäß Fig. 1,
- Fig. 3 bis 5: Querschnitte durch Elektrodenleitungen in weiteren Ausführungsbeispielen, und
- Fig. 6: einen Längsschnitt durch einen Elektrodenkörper in einer weiteren Ausführungsform mit einem schematisch angedeuteten Implantat.

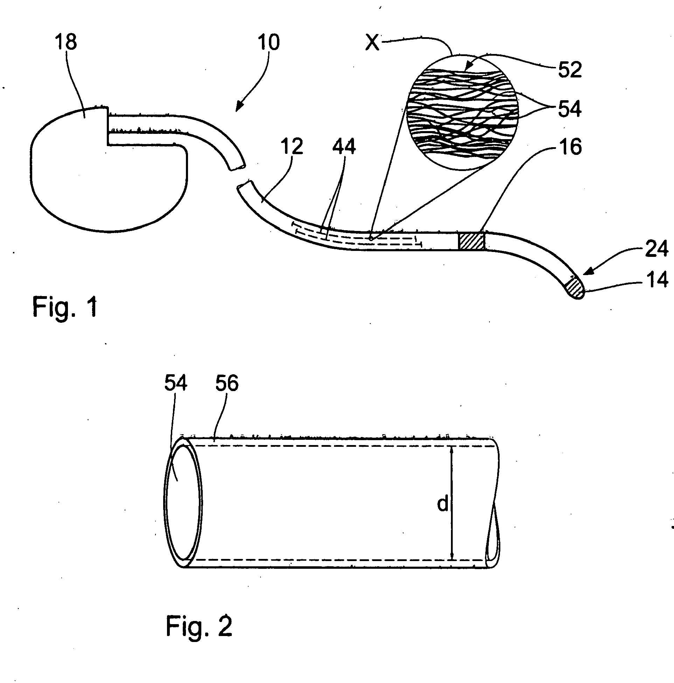

In Fig. 1 ist in schematischer Weise eine Elektrodeneinrichtung in Form eines funktionalen Elektrostimulationsgeräts 10 dargestellt. Dieses besitzt zum einen einen langgestreckten Elektrodenkörper 12, der an seinem distalen Ende 24 zwei Elektroden, nämlich eine Tip-Elektrode 14 und eine Ring-Elektrode 16 trägt. Die Tip-Elektrode 14 und Ring-Elektrode 16 dienen der Detektion von Herzsignalen und der Abgabe von Stimulationsimpulsen an umliegendes Gewebe. Zum anderen weist das Elektrostimulationsgerät 10 ein Gehäuse 18 auf, das die für die Funktionalität des Elektrostimulationsgerätes 10 notwendigen Komponenten, wie einen Impulsgenerator, elektrische Schaltungen und eine Energieversorgung enthält.

Der Elektrodenkörper 12 weist an seinem proximalen, d. h. mit dem Gehäuse 18 verbundenen Ende geeignete, hier nicht näher ersichtliche Strukturen auf, die einen Anschluss an das Gehäuse 18 ermöglichen. Derartige Strukturen sind hinreichend aus dem Stand der Technik bekannt und haben im Zusammenhang mit der vorliegenden Erfindung keine nähere Bedeutung, sodass auf eine ausführliche Beschreibung derselben verzichtet wird.

Die Tip- und Ringelektrode 14, 16 stellen elektrisch leitfähige Strukturelemente dar, die eine Übergangsstelle für elektrische Energie zum Herzgewebe umfassen. Die Ringelektrode 16 kann aus einer Platin-Iridium-Legierung bestehen, während die distale Tipelektrode 14 einen halbkugelförmigen Kopf aus einer Iridium-beschichteten Platin-Iridium-Legierung besteht. Die Elektroden 14, 16 können als Ableit-, Stimulations- oder Messelektroden ausgelegt und in Material, Anzahl, Lage und Geometrie in weitem Maße variiert werden, ohne dass dies für den Gegenstand der vorliegenden Erfindung relevant wäre.

In den Elektrodenkörper 12 sind ferner Elektrodenleitungen 44 (strichliert angedeutet in Fig. 1) eingebettet, die die elektrische Anbindung der Elektroden 14, 16 an die entsprechenden funktionalen Komponenten im Gehäuse 18 herstellen.

Wie aus der mit X bezeichneten, ausschnittsweisen Detailvergrößerung in Fig. 1 sowie aus Fig. 2 hervorgeht, ist die Elektrodenleitung 44 aus einem Faser-Aufbau gebildet, der aus einem Bündel 52 von Einzelfasern 54 aus Kohlenstoff besteht. Diese Einzelfasern 54 verlaufen mit ihrer Haupterstreckungsrichtung im Wesentlichen parallel zur Längsrichtung der Elektrodenleitung 44 und sind - wie Fig. 1 nicht näher entnehmbar ist - in loser, nur mäßig geordneter Lage durch einen Klebstoff gebündelt.

Wie aus Fig. 2 deutlich wird, sind die Einzelfasern 54 mit einer isolierenden Oberflächenschicht 56 versehen, bei der es sich beispielsweise um ein aufgesprühtes, nicht leitfähiges Polymer handeln kann. Die Einzelfaser 54 weist einen Durchmesser d zwischen einigen µm und einigen zehntel Millimeter auf. Mehrere hundert bis mehrere zehntausend isolierte Einzelfasern 54 bilden die Elektrodenleitung 44, sodass diese eine sehr anisotrope Leitfähigkeit aufweist. Die spezifische Leitfähigkeit in Längsrichtung ist um mindestens eine Größenordnung höher als in ihrer Querrichtung. Ein in Längsrichtung der Elektrodenleitung 44 eingespeister Strom kann so relativ ungehindert durch das Faserbündel 52 zur Tip-Elektrode 14 fließen, während ein von außen einwirkendes Magnetfeld erschwerte Induktionsbedingungen vorfindet. Dies trifft insbesondere dann zu, wenn die Elektrodenleitung 44 nicht gestreckt, sondern gekrümmt in einem Magnetfeld liegt.

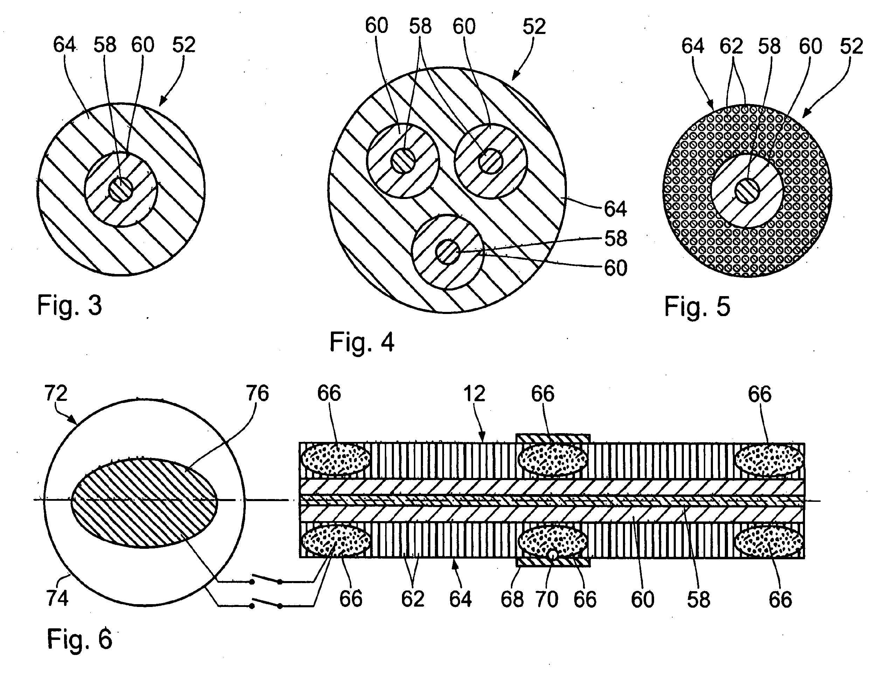

Bei den in Fig. 3 bis 5 gezeigten Ausführungsformen der Erfindung erfolgt die Realisierung eines anisotrop leitenden Faserbündels aus verschiedenartigen Werkstoffen. So kann ein Faserbündel aus einer Kombination von zwei oder mehreren Werkstoffen, wie metallische Fasern, Kohlefasern, leitende Kunststofffasern und halbleitende Fasern, bestehen, wobei bevorzugt Materialien unterschiedlicher Leitfähigkeit kombiniert werden. In allen Fällen handelt es sich um - wie oben erwähnt - isolierte oder nahezu isolierte Einzelfasern. Im Ausführungsbeispiel gemäß Fig. 3 und 4 sind die besser (längs-)leitenden Fasern 58 als Kern des Bündels platziert umgeben von weniger leitenden Fasern 60 mit einer herkömmlichen Isolation. In einem bevorzugten Ausführungsbeispiel nimmt die (Längs-)Leitfähigkeit der ummantelnden Fasern radial graduell ab. In einem weiteren Ausführungsbeispiel mit kombinierten FaserWerkstoffen werden die Fasern höherer Leitfähigkeit unter die anderen Fasern gleichmäßig verteilt. Ein Anwendungsbeispiel ist die Realisierung einer ICD-Elektrodenzuleitung, bei der gut leitende Silberlitzen mit Kohlefasern kombiniert werden.

Eine weitere Ausführungsform der Erfindung gemäß Fig. 5 beruht auf den Einsatz von aktiven und passiven Fasern. Unter aktiven Fasern sind diejenigen zu verstehen, die die Implantatselektronik mit den Elektroden verbinden, d. h. Energie bzw. Signale für die Elektrotherapie bzw. Diagnostik übertragen. Im obigen Text sind stets diese aktiven Fasern 58, 60 gemeint. Davon unterscheiden sich die passiven Fasern 62 dadurch, dass sie für die Elektrotherapie nicht verwendet werden, sondern nur zur Abschirmung von eingestrahlter elektromagnetischer Energie dienen. Die faserartige Struktur soll dabei die Eindringtiefe verringern. Dies ist auf die anisotrop leitende Faserstruktur zurückzuführen. In der bevorzugten Ausführung füllen solche Fasern das Isolationsvolumen möglichst vollständig aus, d. h. der z. B. aus Silikon bestehende E-lektrodenmantel 64 ist ebenfalls von diesen transversal zueinander möglichst isolierten und longitudinal leitenden Fasern 62 durchzogen. Der Isolationsmantel 64 ist dabei nur transversal isolierend, longitudinal ist er leitend. Ziel ist dabei, den gesamten Querschnitt longitudinal leitend zu machen und damit für die induzierten Längsströme geringere Stromdichten zu erreichen. Ebenso entspricht dieser Aufbau hinsichtlich der Längsleitung einem Leiter mit großem Querschnittsradius (es wird das ganze Lumen längsleitend gemacht), der sich bekanntermaßen in elektromagnetischen Wechselfeldern weniger erwärmt.

In einem weiteren Ausführungsbeispiel gemäß Fig. 6 sind diese passiven Fasern 62 an mindestens einem Ende galvanisch miteinander verbunden (z. B. mit einem leitenden Kleber) und verfügen damit über die Möglichkeit, einen implantatsnahen Zusammenschluss-Knoten 66 dauerhaft oder nur in einem bestimmten Betriebsmodus des Implantats 72 an das Implantatsgehäuse 74 und/oder die Implantatselektronik 76 zu konnektieren. In einer erweiterten Ausführung sind mehrere über den Elektrodenkörper 12 verteilte Zusammenschluss-Knoten 66 der passiven Fasern 62 realisiert, die dauerhaft oder nur in einem bestimmten Betriebsmodus des Implantats an leitfähige Strukturen auf dem Mantel des Elektrodenkörpers 12 angeschlossen sind. Dies können beispielsweise Ringelektroden oder Wendeln 68 sein, die galvanischen Kontakt zur Körperflüssigkeit haben und die nicht oder nur temporär nicht für die Therapie verwendet werden. Der Kontaktpunkt 70 kann auch als Schalter realisiert werden, der die Ringelektroden oder Wendeln 68 nur temporär konnektiert.

Ein weiteres Ausführungsbeispiel verwendet als passive Fasern solche, die nicht über die gesamte Elektrodenleitungslänge durchgängig sind. Der Faseraufbau hat dabei eine filzartige Struktur mit Vorzugsrichtung in Längsachse der Elektrodenleitung 44. Dieses gründet auf der Tatsache, dass durch Abstimmung des Durchmesser/Längen-Verhältnisses der Einzelfaser die Reflexionseigenschaften des Abschirmfilzes für die unerwünschte Frequenz der elektromagnetischen Strahlung optimiert werden kann.

## Patentansprüche

1. Elektrodeneinrichtung für die kardiologische oder neurologische Elektrodiagnose und/oder -therapie umfassend
- einen langgestreckten Elektrodenkörper (12),
- mindestens eine Elektrode (14, 16) in der Nähe des distalen Endes (24) des Elektrodenkörpers (12), und
- eine Elektrodenleitung (44) für die elektrische Anbindung der Elektrode (14, 16), wobei die Elektrodenleitung (44) aus einem Faser-Aufbau gebildet ist, der aus einem Bündel (52) von Einzelfasern (54) besteht, wobei das Bündel (52) von Einzelfasern (54) durch lose aneinanderliegende Filamente gebildet ist,
- und wobei die Einzelfasern (54) eine hochohmige bis isolierende Oberflächenschicht (56) aufweisen, welche durch eine Stoffumwandlung des Fasermaterials selbst gebildet ist,
**dadurch gekennzeichnet, dass**
- der Faseraufbau durch die Oberflächenschicht der Fasern eine anisotrope Leitfähigkeit erhält, derart, dass die spezifische Leitfähigkeit der Elektrodenleitung (44) in ihrer Längsrichtung um mindestens eine Größenordnung höher als in ihrer Querrichtung ist.

2. Elektrodeneinrichtung nach einem der vorgenannten Ansprüche, **dadurch gekennzeichnet, dass** die Fasern in sich eine anisotrope, in Längsrichtung signifikant höhere Leitfähigkeit als in Querrichtung aufweisen.

3. Elektrodeneinrichtung nach Anspruch 1, **dadurch gekennzeichnet, dass** die Oberflächenschicht durch eine Oberflächenreaktion, insbesondere Oxidation, des Fasermaterials oder einen Oberflächenumwandlungsprozess während der Faserherstellung gebildet ist.

4. Elektrodeneinrichtung nach einem, der vorgenannten Ansprüche, **dadurch gekennzeichnet, dass** der Faser-Aufbau durch Einzelfasern (54) in einer Anzahl zwischen mehreren zehn und einigen zehntausend, vorzugsweise mehrere hundert bis mehrere tausend, gebildet ist.

5. Elektrodeneinrichtung nach einem der vorgenannten Ansprüche, **dadurch gekennzeichnet, dass** die Einzelfasern (54) aus Kohlenstoff, einem Metall, leitenden Kunststoff oder Halbleitermaterial bestehen.

6. Elektrodeneinrichtung nach einem der vorgenannten Ansprüche, **dadurch gekennzeichnet, dass** das Faserbündel (52) aus einer Kombination von zwei oder mehreren Werkstoffen besteht.

7. Elektrodeneinrichtung nach einem der vorgenannten Ansprüche, **dadurch gekennzeichnet, dass** das Faserbündel (52) mittig gut leitende Fasern (58) hat, die von weniger gut leitenden Fasern (60) umgeben sind, wobei die (Längs-)Leitfähigkeit der ummantelnden Fasern (60) radial graduell abnimmt.

8. Elektrodeneinrichtung nach einem der vorgenannten Ansprüche, **dadurch gekennzeichnet, dass** das Faserbündel (52) aus aktiven (58) und passiven (62) Fasern besteht.

9. Elektrodeneinrichtung nach Anspruch 8, **dadurch gekennzeichnet, dass** die passiven Fasern (62) den Isolationsmantel (64) ausfüllen.

10. Elektrodeneinrichtung nach Anspruch 8 oder 9, **dadurch gekennzeichnet, dass** die passiven Fasern (62) an mindestens einem Ende galvanisch miteinander in einem Zusammenschluss-Knoten (66) verbunden sind.

11. Elektrodeneinrichtung nach einem der vorgenannten Ansprüche, **dadurch gekennzeichnet, dass** der implantatsnahe Zusammenschluss-Knoten (66) dauerhaft oder nur in einem bestimmten Betriebsmodus des Implantats mit dem Implantatsgehäuse (18) und/oder der Implantatselektronik konnektiert ist.

12. Elektrodeneinrichtung nach einem der vorgenannten Ansprüche, **dadurch gekennzeichnet, dass** über den Elektrodenkörper (12) verteilt mehrere solche Zusammenschluss-Knoten (66) der passiven Fasern (62) realisiert sind.

13. Elektrodeneinrichtung nach Anspruch 12, **dadurch gekennzeichnet, dass** die über den Elektrodenkörper (12) verteilten Zusammenschluss-Knoten (66) mit leitfähigen Strukturen auf dem Mantel der Elektrodenleitung (44) elektrisch verbunden sind.

14. Elektrodeneinrichtung nach einem der vorgenannten Ansprüche 8 bis 13, **dadurch gekennzeichnet, dass** die passiven Fasern (62) im Einzelnen nicht über die gesamte Elektrodenteitungslänge durchgängig sind.

## Claims

1. An electrode device for cardiological or neurological electrodiagnosis and/or electrotherapy, comprising
- an elongated electrode body (12),
- at least one electrode (14, 16) near the distal end (24) of the electrode body (12), and
- an electrode line (44) for the electric connection of the electrode (14, 16),
wherein the electrode line (44) is formed from a fiber structure comprising a bundle (52) of individual fibers (54),
wherein the bundle (52) of individual fibers (54) is formed by filaments arranged loosely side by side,
- and wherein the individual fibers (54) have a surface layer (56) which has a high resistance or an insulating effect and is formed by a conversion of the fiber material itself,
**characterized in that**
- the fiber structure has an anisotropic conductivity due to the surface layer of the fibers such that the specific conductivity of the electrode line (44) is higher by at least one order of magnitude in its longitudinal direction than in its transverse direction.

2. The electrode device according to any one of the preceding claims, **characterized in that** the fibers have an anisotropic conductivity which is significantly higher in the longitudinal direction than in the transverse direction.

3. The electrode device according to Claim 1, **characterized in that** the surface layer is formed by a surface reaction, in particular oxidation of the fiber material or a surface transformation process during fiber production.

4. The electrode device according to any one of the preceding claims, **characterized in that** the fiber structure is formed by individual fibers (54) in a number between several tens and a few tens of thousands, preferably several hundred to several thousand.

5. The electrode device according to any one of the preceding claims, **characterized in that** the individual fibers (54) are made of carbon, a metal, conductive plastic or semiconductor material.

6. The electrode device according to any one of the preceding claims, **characterized in that** the fiber bundle (52) consists of a combination of two or more materials.

7. The electrode device according to any one of the preceding claims, **characterized in that** the fiber bundle (52) has fibers (58) that are good conductors at the center of the bundle and are surrounded by fibers (60) that do not such good conductors, wherein the (longitudinal) conductivity of the sheathing fibers (60) decreases gradually in the radial direction.

8. The electrode device according to any one of the preceding claims, **characterized in that** the fiber bundle (52) comprises active fibers (58) and passive fibers (62).

9. The electrode device according to Claim 8, **characterized in that** the passive fibers (62) fill out the insulation jacket (64).

10. The electrode device according to Claim 8 or 9, **characterized in that** the passive fibers (62) are bonded together galvanically in a merger node (66) on at least one end.

11. The electrode device according to any one of the preceding claims, **characterized in that** the merger node (66) near the implant is connected to the implant housing (18) and/or the implant electronics permanently or only in a certain operating mode of the implant.

12. The electrode device according to any one of the preceding claims, **characterized in that** several such merger nodes (66) of the passive fibers (62) are distributed over the electrode body (12).

13. The electrode device according to Claim 12, **characterized in that** the merger nodes (66) distributed over the electrode body (12) are electrically connected to conductive structures on the jacket of the electrode line (44).

14. The electrode device according to any one of the preceding Claims 8 to 13, **characterized in that** the passive fibers (62) as individuals are not continuous over the entire electrode line length.

## Revendications

1. Dispositif à électrodes pour l'électrodiagnostic et/ou l'électrothérapie cardiologique ou neurologique comprenant :
- un corps d'électrode (12) étiré en longueur,
- au moins une électrode (14, 16) à proximité de l'extrémité (24) distale du corps d'électrode (12), et
- une ligne d'électrodes (44) pour la liaison électrique de l'électrode (14, 16), la ligne d'électrodes (44) étant formée à base d'une structure de fibre qui se compose d'un faisceau (52) de fibres individuelles (54),
le faisceau (52) de fibres individuelles (54) étant formé par des filaments disposés les uns contre les autres de façon libre,
et les fibres individuelles (54) présentant une couche superficielle (56) à haute impédance jusqu'à isolante, laquelle est formée par une transformation de matière du matériau fibreux,
**caractérisé en ce que**
- la structure de fibre reçoit par la couche superficielle des fibres une conductibilité anisotrope de telle sorte que la conductivité de la ligne d'électrodes (44) dans son sens longitudinal est supérieure d'au moins un ordre de grandeur à la conductivité dans son sens transversal.

2. Dispositif à électrodes selon l'une quelconque des revendications précédentes, **caractérisé en ce que** les fibres présentent en soi une conductibilité anisotrope, plus élevée dans le sens longitudinal que dans le sens transversal.

3. Dispositif à électrodes selon la revendication 1, **caractérisé en ce que** la couche superficielle est formée par une réaction de surface, en particulier une oxydation du matériau fibreux ou un processus de transformation de surface pendant la fabrication de la fibre.

4. Dispositif à électrodes selon l'une quelconque des revendications précédentes, **caractérisé en ce que** la structure de fibre est formée par les fibres individuelles (54) dans un nombre compris entre plusieurs dizaines et quelques dizaines de milliers, de préférence plusieurs centaines à plusieurs milliers.

5. Dispositif à électrodes selon l'une quelconque des revendications susmentionnées, **caractérisé en ce que** les fibres individuelles (54) sont à base de carbone, d'un métal, de plastique conducteur ou d'un matériau semi-conducteur.

6. Dispositif à électrodes selon l'une quelconque des revendications précédentes, **caractérisé en ce que** le faisceau de fibres (52) est constitué d'une combinaison de deux ou plusieurs matériaux.

7. Dispositif à électrodes selon l'une quelconque des revendications précédentes, **caractérisé en ce que** le faisceau de fibres (52) a au centre des fibres (58) bonnes conductrices, qui sont entourées par des fibres (60) moins bonnes conductrices, la conductibilité (longitudinale) des fibres (60) enveloppantes diminuant graduellement dans le sens radial.

8. Dispositif à électrodes selon l'une quelconque des revendications précédentes, **caractérisé en ce que** le faisceau de fibres (52) est à base de fibres actives (58) et de fibres passives (62).

9. Dispositif à électrodes selon la revendication 8, **caractérisé en ce que** les fibres (62) passives remplissent l'enveloppe d'isolation (64).

10. Dispositif à électrodes selon la revendication 8 ou 9, **caractérisé en ce que** les fibres (62) passives sont reliées par électrolyse les unes avec les autres dans un noeud de regroupement (66) sur au moins une extrémité.

11. Dispositif à électrodes selon l'une quelconque des revendications précédentes, **caractérisé en ce que** le noeud de regroupement (66) proche de l'implant est connecté de façon durable ou seulement dans un mode de service défini de l'implant avec le boîtier d'implant (18) et/ou l'électronique d'implant.

12. Dispositif à électrodes selon l'une quelconque des revendications précédentes, **caractérisé en ce que** plusieurs de tels noeuds de regroupement (66) des fibres (62) passives sont réalisés de façon répartie sur le corps d'électrode (12).

13. Dispositif à électrodes selon la revendication 12, **caractérisé en ce que** les noeuds de regroupement (66) répartis sur le corps d'électrode (12) sont reliés électriquement à des structures conductrices sur l'enveloppe de la ligne d'électrodes (44).

14. Dispositif à électrodes selon l'une quelconque des revendications précédentes 8 à 13, **caractérisé en ce que** les fibres (62) passives ne sont pas continues dans le détail sur l'ensemble de la longueur de la ligne d'électrodes.
